# EUROPÄISCHE PATENTSCHRIFT

(11) **EP 1 749 210 B1**
(45) Veröffentlichungstag und Bekanntmachung des Hinweises auf die Patenterteilung: **13.04.2011**
(21) Anmeldenummer: 05753990.0
(22) Anmeldetag: 25.05.2005
(51) Int. Cl.: G01N 33/50, C12Q 1/68

(54) **ISOLATION ALLERGEN-SPEZIFISCHER IMMUNOGLOBULIN-GENE AUS HUMANEN B-ZELLEN VON ATOPIKERN**
ISOLATION OF ALLERGEN-SPECIFIC IMMUNOGLOBULIN GENES FROM HUMAN B-CELLS FROM ATOPY SUFFERERS
ISOLATION DE GENES D'IMMUNOGLOBULINE SPECIFIQUES D'ALLERGENES, A PARTIR DE LYMPHOCYTES B HUMAINS D'ATOPIQUES

(30) Priorität: 26.05.2004 EP 04012408
(43) Veröffentlichungstag der Anmeldung: 07.02.2007
(73) Patentinhaber: Fraunhofer-Gesellschaft zur Förderung der angewandten Forschung e.V., 80686 München (DE)
(72) Erfinder: ACHATZ, Gernot, A-5020 Salzburg (AT); HUHN, Michael, 51375 Leverkusen (DE); FISCHER, Rainer, 52156 Monschau (DE); FERREIRA, Fatima, A-5020 Salzburg (AT); LUGER, Elke, A-4614 Marchtrenk (AT); STÖCKER, Michael, 52074 Aachen (DE); BARTH, Stefan, 52159 Roetgen (DE); KLOCKENBRING, Torsten, 53121 Bonn (DE)
(74) Vertreter: von Kreisler Selting Werner
(86) Internationale Anmeldenummer: PCT/EP2005/052398
(87) Internationale Veröffentlichungsnummer: WO 2005/116645

(56) Entgegenhaltungen:
- WO-A-92/01047
- LIEBY PATRICIA ET AL: "The clonal analysis of anticardiolipin antibodies in a single patient with primary antiphospholipid syndrome reveals an extreme antibody heterogeneity" BLOOD, Bd. 97, Nr. 12, 15. Juni 2001 (2001-06-15), Seiten 3820-3828, XP002288754 ISSN: 0006-4971 in der Anmeldung erwähnt
- WANG X ET AL: "Human immunoglobulin variable region gene analysis by single cell RT-PCR" JOURNAL OF IMMUNOLOGICAL METHODS, ELSEVIER SCIENCE PUBLISHERS B.V.,AMSTERDAM, NL, Bd. 244, Nr. 1-2, 20. Oktober 2000 (2000-10-20), Seiten 217-225, XP004218461 ISSN: 0022-1759 in der Anmeldung erwähnt
- DE WILDT R M T ET AL: "A new method for the analysis and production of monoclonal antibody fragments originating from single human B cells" JOURNAL OF IMMUNOLOGICAL METHODS, ELSEVIER SCIENCE PUBLISHERS B.V.,AMSTERDAM, NL, Bd. 207, Nr. 1, 22. August 1997 (1997-08-22), Seiten 61-67, XP004093141 ISSN: 0022-1759
- FEUCHTENBERGER M ET AL: "Semiquantitative and qualitative assessment of B-lymphocyte VH repertoire by a fluorescent multiplex PCR" JOURNAL OF IMMUNOLOGICAL METHODS, ELSEVIER SCIENCE PUBLISHERS B.V.,AMSTERDAM, NL, Bd. 276, Nr. 1-2, 1. Mai 2003 (2003-05-01), Seiten 121-127, XP004422646 ISSN: 0022-1759
- RASSENTI LAURA Z ET AL: "Analysis of immunoglobulin V-H gene repertoire by an anchored PCR-ELISA" 1995, ANNALS OF THE NEW YORK ACADEMY OF SCIENCES; IMMUNOGLOBULIN GENE EXPRESSION IN DEVELOPMENT AND DISEASE NEW YORK ACADEMY OF SCIENCES {A}, 2 EAST 63RD STREET, NEW YORK, NEW YORK 10021, USA SERIES : ANNALS OF THE NEW YORK ACADEMY OF SCIENCES (ISSN 0077-8 , CONFERENCE; MONTREAL, QUEBEC, CANADA; JULY 13-17, 1994 , XP008032812 ISSN: 0-89766-934-7 0-89766-933-9 Seite 464, Absatz 2 - Absatz 3 Abbildung 1 Seite 471, Zeile 26 - Zeile 29
- HUSE W D ET AL: "GENERATION OF A LARGE COMBINATORIAL LIBRARY OF THE IMMUNOGLOBULIN REPERTOIRE IN PHAGE LAMBDA" SCIENCE, AMERICAN ASSOCIATION FOR THE ADVANCEMENT OF SCIENCE,, US, Bd. 246, Nr. 4935, 8. Dezember 1989 (1989-12-08), Seiten 1275-1281, XP000083689 ISSN: 0036-8075
- WARD E S ET AL: "BINDING ACTIVITIES OF A REOPERTOIRE OF SINGLE IMMUNOGLOBULIN VARIABLE DOMAINS SECRETED FROM ESCHERICHIA COLI" NATURE, MACMILLAN JOURNALS LTD. LONDON, GB, Bd. 341, Nr. 6242, 12. Oktober 1989 (1989-10-12), Seiten 544-546, XP000086104 ISSN: 0028-0836

## Beschreibung

Die Erfindung betrifft ein Verfahren zur Bestimmung des immunologischen Reaktionsprofils eines Organismus. Weiterer Bestandteil der Erfindung sind die RT- und PCR Produkte erhältlich durch das oben genannte Verfahren, wie auch die Vektoren, die diese RT- und PCR Produkte enthalten. Die Erfindung betrifft darüber hinaus die Verwendung dieser Vektoren zur Herstellung von rekombinanten Antikörpern, sowie die rekombinanten Antikörper, als auch die Verwendung dieser rekombinanten Antikörper in dem oben genannten Verfahren zur Analyse ihrer Bindungsaktivität.

### Hintergrund der Erfindung

Charakteristisch für atopische Erkrankungen sind Überempfindlichkeitsreaktionen vom Typ **I.** Pathopysiologisch wird die Erkrankung auf Störungen immunlogischer Toleranzmechanismen zurückgeführt, die im einzelnen noch nicht geklärt sind. Dabei richten sich die zu Grunde liegende Immunreaktionen zumeist gegen harmlose Antigene, die von Gesunden toleriert werden. Diese Entzündungsreaktionen werden durch Immunglobuline der Klasse E vermittelt. Die IgE Moleküle werden durch Mastzellen und basophile Granulozyten über Oberflächenrezeptoren gebunden. Kommt es bei Antigenkontakt zu Kreuzvernetzungen von IgE/IgE Rezeptorkomplexen, so werden bei den betreffenden Zellen Prozesse induziert, die zu einer Sekretion von Entzündungsmediatoren führen. Dies ist der Ausgangspunkt für die vielfältige Symptomatik atopischer Erkrankungen.

Eine humorale Immunantwort beginnt mit einer IgM dominierten primären Antikörperantwort. Später spezifiziert sich das Antikörperrepertoire, durch eine Switch-Rekombination zu den Antikörper Isotypen IgG, IgA oder IgE. Diese Antikörperklassen werden später auch für "Memory-Antikörper" herangezogen. Charakteristisch für die Antikörper der sekundären Immunantwort ist, dass sie eine höhere Affinität zu einem bestimmten Antigen aufweisen. Das Phänomen das dieser Entwicklung zugrunde liegt bezeichnet man als *"Affinitätsreifung des Antikörper Repertoires".* Es wird durch somatische Mutationen in den hypervariablen Regionen der variablen Bereiche der schweren und leichten Antikörperketten erreicht und findet in den Keimzentren von lymphatischen Organen statt. Die Produzenten der Immunglobuline sind B-Lymphozyten, deren Reifung, Differenzierung und klonale Expansion wesentlich durch den wechselseitigen Kontakt mit antigenpräsentierenden Zellen und T- Helferzellen (Th-Zellen) bestimmt werden. Dabei exprimiert jeder B-Zellklon nur eine, genetisch einmalige Variante eines Immunglobulins. Die Variabilität unterschiedlicher Bindungsspezifitäten ist somit durch das Repertoire unterschiedlicher klonaler Ursprungszellen determiniert. Das Verhältnis der Größe der einzelnen Klone zueinander ist durch die dynamische Anpassung an die aktuellen Erfordernisse geprägt. Die spezifische Bindungsaktivität gegenüber Antigenen ist über die Konstitution der variablen Regionen der leichten und schweren Ketten eines Antikörpers festgelegt.

An der Entwicklung von IgE-sezernierenden Plasmazellen haben Th-Zellen des Typs 2 bedeutenden Einfluss. Die Ausrichtung immunologischer Prozesse auf ein bestimmtes Antigen wird durch hoch spezifische Oberflächenrezeptoren (B-Zellrezeptor, T -Zellrezeptor) auf den B- und Th-Lymphozyten gewährleistet, die den initialen Kontakt beider Zelltypen nur in Gegenwart eines Antigens ermöglicht, für das beide über einen spezifischen Rezeptor verfügen müssen. Diese Antigen-spezifische Orientierung der Immunreaktion kann bei hohen IgE-Titern verloren gehen, wenn B-Zellen unterschiedlichster Spezifität über den niedrig affinen Rezeptor für IgE (CD23) lösliche Immunglobuline dieser Klassen binden und damit neben ihrer ursprünglichen Spezifität, die durch den membranständigen B-Zellrezeptor repräsentiert wird -IgE-spezifische Antigene erkennen, internalisieren und über MHC II-Moleküle mit Th2- Zellen in antigenspezifischen Kontakt zu treten. In der Folge entstehen so weitere IgEsezernierende Plasmazellen, die das hypersensitive Antigenspektrum extrem erweitern können [1].

Die Beteiligung einzelner Immunglobulinklassen am atopischen Geschehen ist zwar beschrieben [2], die funktionellen Bezüge unterschiedlicher Antikörperklassen innerhalb des Immunsystems, ihre Einflüsse auf das Gesamtgefüge z.B. im Rahmen physiologischer und pathophysiologischer Prozesse (wie den atopischen Erkrankungen, darüber hinaus aber auch autoreaktive Reaktionen, Sepsis, Toleranz, und Anergie steuernde Vorgänge) sind dagegen nur unzureichend verstanden. Tiefergehende Erkenntnisse dieser Prozesse lassen sich aus differenzierten Reaktionsprofilen der am Geschehen beteiligten B-Zellklone ableiten, die den Einfluss auf das Spektrum des antigenspezifischen V-Gen- bzw. Antikörperrepertoirs und Isotypmusters im funktionellen Bezug zum immunphysiologischen bzw. pathophysiologischen Status abbilden können. Bislang stellt die enorme Vielfalt des Antikörperrepertoires eine entscheidende Hürde für die Erstellung zeitlich eng auflösender, repräsentativer Reaktionsprofile dar.

Begrenzte Antigenspezifische Reaktionsprofile von Antikörpern werden auf der Basis von Serumanalysen durchgeführt. Dabei kann das Blutserum auf Reaktivität gegen ca. 600 verschiedene Allergene hin untersucht werden. Diese Methodik erlaubt aber keine Aussagen über nicht sezernierende B-Zellen, die durch suppressive Einflüsse an der Sezernierung von Antikörpern gehemmt sind. Es werden somit nicht alle potentiellen reaktiven Zellen erfasst, und es ist mit dieser Methode nicht möglich die Prädisposition zu Immunerkrankungen zu bestimmen.

Weiterhin ist es möglich das Antigen-spezifische Reaktionsprofil direkt am Patienten, *in vivo*, durch Haut-Prick-, Intrakutan- und Provokationstests zu bestimmen. Nachteile dieser Methoden sind, dass sie den Patienten belasten und bei hochgradigen Allergikern, nur beschränkt anwendbar sind. Antigenspezifische V-Gen Profile auf Basis von Einzelzellanalysen sind ebenfalls beschrieben [3], [4]. Diese Ansätze sind auf eine repräsentative Darstellung des Antigen-spezifischen V-Gen Repertoires ausgelegt, aber in dieser Form nicht für eine umfassende isotypenspezifische Analyse der V-Gen Repertoires genutzt worden.

In der letzten Zeit gelang es auch im Modellsystem Maus, B-Zellen zu isolieren, die dem Affinitätsreifungsprozess (somatische Hypermutation) unterliegen. Ein Protokoll wurde entwickelt, das die nachfolgende Isolierung von B-Zellen mittels FACS ermöglichte. Aus den Hapten- bzw. Allergenspezifischen Zellen wurde RNA isoliert und in Folge in cDNA revers transkribiert. Aus der cDNA konnten anschließend Allergen/Hapten spezifische schwere Ketten von Immunglobulinen mittels RT-PCR isolieren werden. Die Sequenzen wurden dann einer intensiven Computeranalyse unterzogen. Folgende Ergebnisse wurden erzielt:
- Ohne jeden Zweifel kann die IgE-Antwort "reifen".
- Die somatische Diversität ist von Isotyp zu Isotyp unterschiedlich.
- Der Antigenrezeptor ist für die Selektion von hoch affinen Antikörpern notwendig.
- Der Antigenrezeptor hat für die Regulation des somatischen Mutationsprozesses keine Funktion.
- Je kürzer die CDR3 Region, je affiner der Antikörper gegen das Hapten.
- IgE bildet keine "long lived"-Plasmazellen.
- Die IgE-Antwort ist kurzlebig.
- Der zytoplasmatische "tail" scheint in geeignetes Target für eine Allergietherapie zu sein.

Die Ergebnisse dieses Teilprojektes wurden im "European Journal of Immunology" publiziert [5].

Bedingt durch die enorme Vielfalt des Antikörperrepertoires war es bislang somit problematisch, bzw. unmöglich, ein umfassendes Antigen-spezifischen Reaktionsprofil eines Organismus zu bestimmen. Insbesondere fehlte ein für den Patienten wenig belastendes und risikoarmes Verfahren zur Beurteilung seines allergischen (antigen-spezifischen) Reaktionsprofils. Überraschenderweise bietet die vorliegenden Erfindung eine Lösung dieses Problems an. Der Erfindungsgegenstand der vorliegenden Anmeldung erlaubt auf genetischer Ebene die Bestimmung des Antigen-spezifischen Reaktionsprofils eines Organismus. Überrachsenderweise ist es so möglich, alle B-Zell-vermittelten Antigen-spezifische Reaktionen zu erfassen, insbesondere auch solche bei denen die Sezernierungsaktivität der B-Zellen durch suppressive Einflüsse gehemmt ist.

### Kurzbeschreibung der Figuren

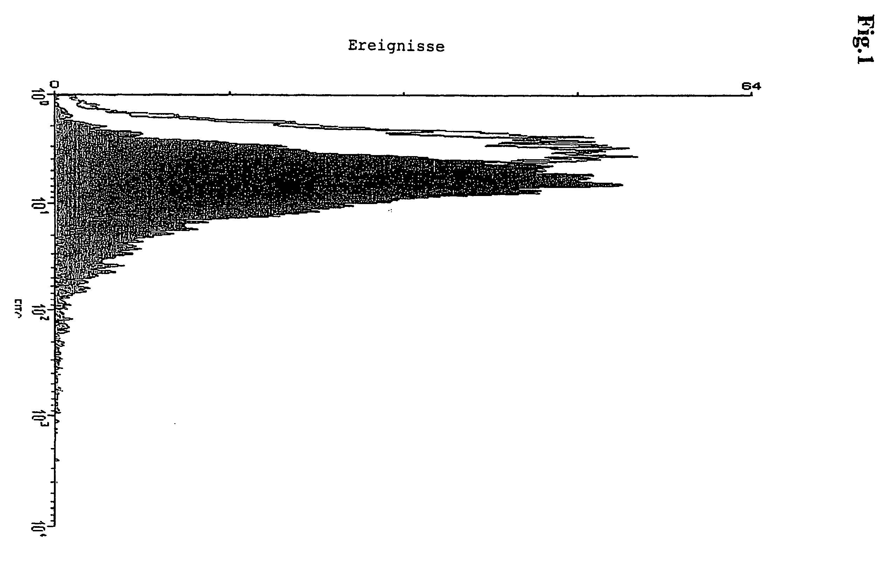

Figur 1: FACS Analyse EBV transformierter humaner B-Zellen, die gegen das Lieschgras-Allergen Phl pVb selektioniert wurden. Gezeigt ist die spezifische Bindung an LPV-ETA' durch ein EBV transformierte B-Zelllinie (gefüllte Kurve), die aus einem Antigen spezifischen Selektionsverfahren hervorgegangen ist. LPV-ETA' stellt ein Fusionsproteinaus dem rekombinanten Allergen Phl p5b und dem modifizierten Enterotoxin A aus *Staphylococcus aureus* dar. Bindungsaktivitäten, die sich gegen das Toxinfragment richten, wurden mit einem Kontrollantigen HAI-ETA' untersucht, einem Fusionsprotein aus einem Antikörperfragment und der oben erwähnten Enterotoxin A Domäne (offene Kurve). Unspezifische Reaktionen die als Hintergrund der Messungen durch den Einsatz sekundärer Nachweiskomponenten auftreten, sind mit der offenen Kurve dargestellt.
Figur 2: Die Darstellung zeigt mit einer FACS Analyse den Vergleich der Bindungsaktivität der Phl p5b spezifischen EBV transformierten Zelllinie (gefüllte Kurve) mit einer EBV transformierten Zelllinie ohne Bezug zu Phlp5b (offene Kurve). Unspezifische Reaktionen die als Hintergrund der Messungen durch den Einsatz sekundärer Nachweiskomponenten auftreten, sind mit der offenen Kurve dargestellt.
Figur 3: (a) und (b) zeigen die Anzahl der B-Zellcluster nach EBV-Transformation in Bezug zur Verdünnungsreihe der EBV-Überstände. Berechnete Korrelationskoeffizienten: (a) = -0,95; (b) = -0,96, n=3.
Figur 4: Immunklassen-spezifischer ELISA mit Überständen EBV transformierter B-Zellen. Der Nachweis wurde für 10 Subkulturen geführt. Die Werte beruhen auf kontrollbereinigten Doppelbestimmungen mit Ansätzen ohne primäre Nachweiskomponente. Als Negativkontrolle diente ein Ansatz gegen Kulturmedium.

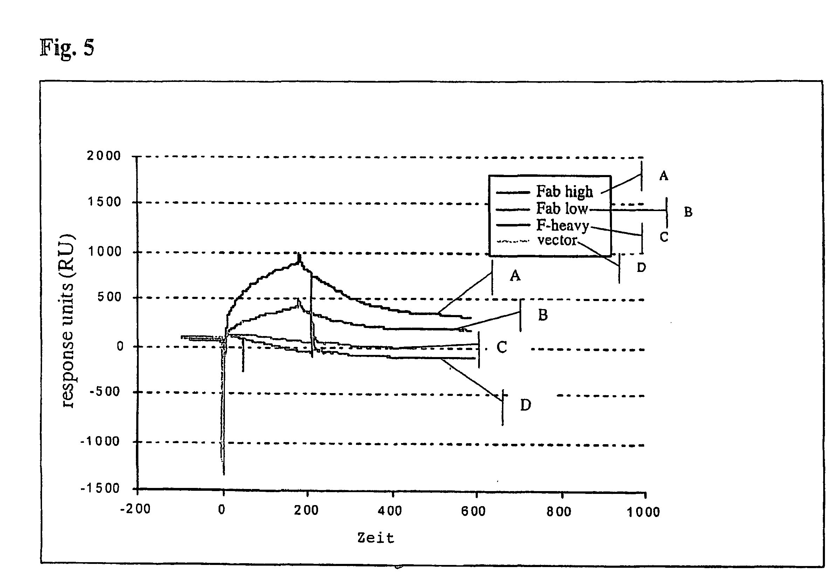

Figur 5: Affinitätsmessung reklonierter und exprimierter anti Betvl spezifischer Fab-Fragmente mittels der BIACORE- Methode

### Detaillierte Beschreibung

Ziel der Erfindung ist es, das individuelle Repertoire Ig-vermittelter Bindungsaktivitäten von Organismen, insbesondere von Menschen mit atopischen Erkrankungen und/oder Autoimmunerkrankungen, anhand rekombinanter Ig-spezifischer V Gene umfassend darzustellen. Dabei sollen alle Immunglobulinklassen berücksichtigt werden, da neben den IgEspezifischen Reaktionen Bindungen von Antikörpern anderer ImmunglobulinKlassen am selben Antigen wichtige Regulative für das entzündliche Geschehen darstellen können.

Nachstehende Definitionen sind auf die gesamte vorliegenden Anmeldung anzuwenden.

Organismus: unter diesem Begriff werden alle Lebewesen verstanden, die von Immunfunktionsstörungen, wie atopischen Erkrankungen (Allergien) und/oder Autoimmunerkrankungen betroffen sein können. Insbesondere sind dies Säugetiere, einschließlich des Menschen.

Immunologisches Reaktionsprofil: dieser Begriff beschreibt die Fähigkeit eines Organismus gegen ein Antigen spezifische Antikörper zu bilden.

Mit dem vorliegenden *in vitro* Verfahren zur Bestimmung des immunologischen Reaktionsprofils eines Organismus kann in kurzer Zeit ein individuelles Antigen spezifisches V-Gen Repertoire dargestellt und des Ig Isotyp spezifisch differenziert werden, ohne dabei den Organismus zu belasten. Damit ergibt sich die Möglichkeit Krankheitsverläufe - vor allem solche, die durch Störungen B-Zell vermittelter Funktionen beruhen - anhand von Antigen und Isotyp spezifischen Reaktionsprofilen zu erfassen und zu bewerten:

Der Erfindungsgegenstand ist ein Verfahren zur Bestimmung des immunologischen Reaktionsprofils eines Organismus, der die folgenden Schritte umfasst: (a) Isolation, einmalig oder mehrmalig, einer Fraktion aus Körperflüssigkeiten und/oder Geweben, die B-Zellen enthält; (b) Isolation von einzelnen B-Zellen oder deren genetischen Materials und Ablage von einer oder mehreren B-Zellen, oder deren genetischen Materials, in ein Reaktionsgefäß; (c) Amplifikation des die variablen Regionen eines von einer isolierten B-Zelle exprimierten Antikörpers codierenden genetischen Materials; (d) Herstellung von rekombinanten Antikörpern durch Exprimierung der klonierten Amplifikationsprodukte und (e) Bestimmung der Bindung des rekombinanten Antikörpers an rekombinante und/oder native Antigene. Dieses Verfahren ermöglicht die Bestimmung des immunologischen Reaktionsprofils von Organismen, insbesondere Personen, deren Immunsystem nicht offensichtlich gestört ist, insbesondere zur Bestimmung des Potentials einer Prädisposition für atopische Erkrankungen oder Antikörper vermittelte Autoimmunerkrankungen. Ebenso eignet sich dieses Verfahren zur Bestimmung des immunologischen Reaktionsprofils von Organismen, wie Personen, mit Immunfunktionsstörungen. Insbesondere eignet sich das erfindungsgemäße Verfahren zur Bestimmung des immunologischen Reaktionsprofils bei Immunfunktionsstörungen die folgende Erkrankungen umfassen: Allergien, atopische Erkrankungen, Antikörper-vermittelte Autoimmunerkrankungen wie Antiglomeruläre Basalmembrankrankheit; Arthritis, rheumatoide; Autoimmunkrankheiten des Nervensystems, z.B. Multiple Sklerose; Diabetes mellitus, insulinabhängiger; Lupus erythematodes, systemischer; Pemphigus; Addison-Krankheit; Anämie, autoimmunhämolytische; Antiphospholipid-Syndrom; Dermatitis, herpetiformis Duhring; Glomerulonephritis, IgA-; Glomerulonephritis, membranöse; Goodpasture-Syndrom; Graves-Krankheit; Lambert-Eaton-Myasthenie-Syndrom; Ophthalmie, sympathische; Pemphigoid, bullöses; Polyendokrinopathien, Autoimmun-; Purpura, thrombozytopenische, idiopathische; Reiter-Krankheit; Thyreoiditis, Autoimmun; Hashimoto Thyreoiditis; primäres Myxödem; Thyreotoxikose (Basedow Krankheit); Myasthenia gravis; Anämie, perniziöse; Leukopenie, autoimmune; Thrombozytopenie, idiopathische; Zirrhose, primäre, biliäre; Autoimmunhepatitis; Colitis ulcerosa; Sjörgen Syndrom; rheumatisches Fieber; Dermatomyositis Polymyositis; Sklerose, progressive, systemische; Granulomatose, Wegner; Panateriitis nodosa; Periateriitis nodosa; und Hypersensitivitätsangiitis etc. Bei dem erfindungsgemäßen Verfahren wird anhand des genetischen Materials der B-Zellen das immunologische Reaktionsprofil ermittelt und quantitativ und qualitativ verglichen mit dem Reaktionsprofil aus dem Serum. Bei dem erfindungsgemäßen Verfahren wird die B-Zell-haltige Fraktion aus peripheren mononukleären Zellen über Dichtezentrifugation aus Blutproben isoliert. Die Isolation einzelner B-Zellen umfasst bevorzugt die folgenden Schritte: (a) die Markierung der B-Zellen mittels Fluorochrom gekoppelter Antikörper, bevorzugt mit Antikörpern spezifisch für CD19, CD85 und CD138, CD19, CD85, CD 138, CD10, CD20, CD21, CD22, CD23, CD24, CD27, CD37, CD38, CD39, CD40, CD72, CD73, CD74, CDw75, CDw76, CD77, CDw78, CD79, CD80, CD81, CD82, CD83, CDw84, CD85 und/oder CD86, usw.; (b) die durchflusszytometrische Selektion der markierten B-Zellen; und (c) die Ablage von einer oder mehreren B-Zellen in ein Reaktionsgefäß. Bei dem erfindungsgemäßen Verfahren werden die B-Zellen nach dem Isolierungsschritt und der Einzelzellablage durch EBV Transformation immortalisiert. Es ist auch möglich die B-Zellen nach dem Isolierungsschritt und der Einzellzellablage mit Myelomzellen zu fusionieren.

Das erfindungsgemäße Verfahren zur Bestimmung des immunologischen Reaktionsprofils umfasst folgende Amplifikationsschritte: (a) cDNA generierende RT Reaktionen; (b) PCR Reaktionen mit der cDNA als Matrize und (c) nested PCR Reaktionen mit den Amplifikationsprodukten aus der PCR Reaktion als Matrize. Dabei werden in den RT Reaktionen Primer eingesetzt, die sequenzkomplementär zu den konstanten Regionen der leichten und schweren Ketten sind, und es werden als Amplifikationsprodukt cDNAs gewonnen. In den PCR Reaktionen wird die cDNA als Matrize eingesetzt und degenerierte Primer, die sequenzkomplementär zu den variablen Regionen im framework 1 Bereich der 6 Genfamilien der schweren Ketten sind, und/oder es werden in den PCR Reaktionen die cDNA als Matrize eingesetzt und degenerierte Primer für die 4 Genfamilien der κ- Ketten, und/oder es werden in den PCR Reaktionen die cDNA als Matrize eingesetzt, und degenerierte Primer für die konstanten Regionen der ε- γ- und der κ- Ketten. In den "nested" PCR Reaktionen werden die Amplifikationsprodukte aus der PCR Reaktion als Matrize eingesetzt und 5' Primer, die sequenzkomplementär zu den ersten 6 Codons der V_{H}- oder der V_{L} Regionen sind, und 3' Primern, die sequenzkomplementär zu den "genesteten" konstanten Regionen der κ-, λ-, α1-, α2-, γ1-, γ2-, γ3-, γ4-, µ- und ε- Ketten sind. In dem erfindungsgemäßen Verfahren werden die Amplifikationsprodukte aus den Reaktionsgemischen, die Amplifikate der V_{L} und der V_{H} Regionen enthalten, in einen Vektor kloniert und durch Exprimierung der Vektoren in bakteriellen und/oder eukaryontischen Expressionssystemen rekombinante Antikörper gewonnen. Es werden auch die Vektoren des erfindungsgemäßen Verfahren, die die Amplifikationsprodukte enthalten, beansprucht. Ebenso wird die Verwendung der erfindungsgemäßen Amplifikationsprodukte zur Herstellung von Expressionsvektoren beansprucht. Weiterhin wird die Verwendung der Vektoren zur Herstellung von rekombinanten Antikörpern beansprucht, sowie die so erhältlichen rekombinanten Antikörper. Das erfindungsgemäße Verfahren umfasst auch die Bestimmung der Bindung der rekombinanten Antikörper an Antigene durch automatisierte Verfahren, insbesondere durch festphasengekoppelte Bindungsassays, Durchflußzytometrie, Resonanzspektrometrie und/oder Chip Arrays mit rekombinanten und nativem Antigenen in nativer oder denaturierter Form. Es wird auch die Verwendung der rekombinanten Antikörper zur Bestimmung ihrer Bindungsaktivitäten für rekombinante Antigene beansprucht.

Ausgehend von peripheren Blutproben umfasst die vorliegende Erfindung zur Bestimmung des immunologischen Reaktionsprofils eines Organismus nachfolgende Schritte: (a) die Isolation, einmalig oder mehrmalig, von B-Zellen sowie deren Sortierung und Einzelzellablage und die Immortalisierung der B-Zellen durch EBV Transformation bzw. die Heteromylomfusion von der B-Zellen mit der B-Lymphom Zelllinie KGH6/B5; (b) die Isolation des genetischen Materials der einzelnen oder mehrerer B-Zellen; (c) die spezifische Amplifikation der variablen Regionen leichter In- und λ-ketten sowie der variablen Regionen der schweren Iga1, Igo2, Igγ1, Igγ2a, Igγ2b, Ig3, Igγ4, Igµ und Igε-Kette mittels verschiedener PCR Reaktionen, einschließlich einer Einzelzell "nested" RT- PCR, (d) die funktionelle Expression der Amplifikate in einem bakteriellen Expressionssystem zur rekombinanten Herstellung von Antikörpern und (e) die automatisierte qualitative und quantitative Analyse der Bindungsaktivitäten rekombinanter Antikörper durch festphasengekoppelte Bindungsassays, Durchflußzytometrie, Resonanzspektrometrie, Chip Arrays mit rekombinanten und nativem Antigenen in nativer oder rekombinanter Form, um so auch nichtproteinogene, aus post-translationalen Modifikationen herrührende Epitopstrukturen erfassen zu können.

### Detaillierte Beschreibung

Bei der vorliegenden Erfindung handelt es sich um ein Verfahren zur Bestimmung des immunologischen Reaktionsprofils eines Organismus, das mehrere Schritte umfasst. Als erstes werden die allergenspezifischen Plasmaund Gedächtnis-B-Zellen aus einer Körperflüssigkeit und/oder Geweben, die B-Zellen enthalten, angereichert. Die Anreicherung dieser Zellen ist beispielsweise durch Dichtezentrifugation von peripheren mononukleären Zellen aus Blutproben möglich. Tote Zellpopulationen werden mittels Propidiumiodid ausgeschieden. Die allergenspezifische Gedächtniszellpopulation beträgt in der FACS Analyse im Durchschnitt 1,2%. Die Gedächtnis-B-Zellpopulation werden, wie auch im Methodenteil beschrieben, als Einzelzellen sortiert. Die Isolation umfasst drei Schritte, nämlich (a) die Markierung der B-Zellen mittels Fluorochrom gekoppelter Antikörper, wobei Antikörper spezifisch für CD19, CD 138, CD10, CD20, CD21, CD22, CD23, CD24, CD27, CD37, CD38, CD39, CD40, CD72, CD73, CD74, CDw75, CDw76, CD77, CDw78, CD79, CD80, CD81, CD82, CD83, CDw84, CD85 und/oder CD86, usw., verwendet werden können; (b) die durchflusszytometrische Selektion der markierten B-Zellen; und (c) die Ablage von einer oder mehreren Zellen in eine Reaktionsgefäß. Die abgelegten Zellen können durch Transformation mit dem Eppstein Barr Virus (EBV) immortalisiert werden; es ist auch möglich, die isolierten/abgelegten/sortierten B-Zellen mit Myelomzellen zu fusionieren. Als zweiter Schritt schließt sich die RNA-Isolierung aus den sortierten Zellen an. Als dritter Schritt erfolgt die Amplifikation des genetischen Materials, das die variablen Regionen des von der B-Zelle exprimierten Antikörpers codiert. Es wird zunächst eine RT-PCR zur Amplifizierung der Immunglobulin schweren und leichten Ketten durchgeführt. Dabei werden zur Gewinnung der cDNA-Amplifikationsprodukte Primer eingesetzt, die sequenzkomplementär zu den konstanten Regionen der leichten und schweren Ketten sind. Insbesondere werden für die RT Reaktionen folgende Primer eingesetzt: (a) leichte Kette κ:5' AAC AGA GGC AGT TCC AGA; (b) leichte Kette λ: 5' TGTGGC CTT GTT GGC TTG; (c) schwere Kette α: 5' CTTGCA GAC ACT TGG TGT TCG TGC; (d) schwere Kette γ1 und γ3: 5' AGG GYG CCA GGG GGA A; (e) schwere Kette γ2: 5' TTT ACC CRG AGA CAG GGA GAG GC; (f) schwere Kette γ4: 5' TTT ACC CRG AGA CAG GGA GAG GC; (g) schwere Kette µ: 5'GCA GGA GAC GAG GGG GA; und (h) schwere Kette ε: 5' TTT ACC GGG ATT TAC AGA CAC. Die schweren Ketten werden auf alle Isotypen hin analysiert. Bisher konnten allergenspezifische Varianten diverser Isotypen amplifiziert und exprimiert werden. Naturgemäß findet sich allerdings die Ig-Klasse gamma 1 am häufigsten. Es wird darauf hingewiesen, dass nicht automatisch dieselben variablen Gene für alle Isotypen verwendet werden (siehe [14]). Vielmehr findet sich auch hier eine Isotypen spezifische Verwendung von V- Genklassen. Die durch die RT-PCR gewonnen cDNA Fragmente werden als Matrizen in PCR Reaktionen eingesetzt, die degenerierte Primer enthalten, die sequenzkomplementär zu den variablen Regionen im framework 1 Bereich der sechs Genfamilien der schweren Ketten sind, und/oder sequenzkomplementär für die vier Genfamilien der κ-Ketten sind, und/oder sequenzkomplementär für die drei Genfamilien der λ-Ketten sind, und/oder mit degenerierten Primern für die konstanten Regionen der ε- γ- und der κ- Ketten. Insbesondere werden für die variable Region der leichten κ-Ketten nachfolgende "sense" Primer eingesetzt: hVkI Back 5' GAC MTC VWG HTS ACC CAG TCT CC; hVkII Back 5' GAC CTC CAG HTG ACC CAR WSY CC; hVkIII Back 5' GAC CTC CAR HTS ASK CAG TCT CC; hVkIV Back 5' GAK VTY GTG ATG ACY CAG WCT CC; hVkV Back 5' GAC ATC SWG ATG ACC MAG TCT CC; hVkVI Back 5' GAC CTC GTG HTG ACB CAG DSY CC; hVkVII Back 5' GAC ATS VWG CTC ACS CAG TCT CC. Als anti-"sense" Primer werden für die variable Region der leichten κ-Ketten nachfolgende Primer eingesetzt: hVkI For 5'TCG TTT GAT CTC CAS YYK KGT CC; hVkII For 5' TCG TTT RAT YAG T'AC CYK KGT CC; hVkIII For 5' TCG TTT GAY HTS CAS CTT KGT CC. Für die variablen Regionen der leichten λ-Kette werden nachfolgende "sense" Primer eingesetzt: hVλI Back 5' CAG TCT SWG CTG ACK CAG CCR CC; hVλII Back 5' CAG TCT SMG CTG ACT CAG CCW SS; HVλII Back 5'CAS GYT WTA YTG ACT CAA YCG CC; hVλIV Back 5' TCC TMT GWG CTG ACW CAR CCA CC; HVλV Back 5'TCK TMT GAR CTG ACT CAR GAC CC; hVλVI Back 5' CAG TCT GTG CTG ACT CAG SMD SS. Als anti-`sense" Primer für die variablen Regionen der λ-Kette werden eingesetzt: hVλI For 5' ACCKAG RAC GGT SAS CTB GGT CC; hVλII For 5'ACY TAR GAC GGT SAV YTT GGT CC; hVλIII For 5'ACC TAR RAC GGT SAV CTK GGT CC. Weiterhin wurden als Immunglobulin-spezifischen Primer ("sense") für die schweren Ketten eingesetzt: hVHI 5'CAG GTG CAG CTG SWG SAR TCK GG; hVHII sense 5' CAG STG CAK CTG CAG GAG TCS GG; HvhIII sense 5'CAG GTG SAG CTG SWG SAG TCH GG; HvhIV sense 5'GAG GTG CAG CTR CAK SAG TSG GG; HvhV sense 5'GAG GTG CAR CTG KTG SAG TCN GS; HvhVI sense 5'GAG GTG CGR CTG GTG SAG WSK GG; HvhVII sense 5' CAG GTS AAS YTA AGG GAG TCT GG. Als anti-"sense" Immunglobulin-spezifische Primer für schweren Ketten werden eingesetzt: IgA1 5'GAA GAC CTT GGG GCT GGT CGG GGA TG; IgA2 5' ATG CGA CGA CCA CGT TCC CAT CTT G; IgE 5'GTT CTG GAT CAG GCA GGC GAG GGT GC; IgG1 5'CTC GAT GGGGGC TGG GAG GGC TTT G; IgG2 5'CCT GGT GCA CAA CGG TGA GGA CG; IgG3 5' GTC CGG GAA ATC ATA AGG GTA TC; IgG4 5' GAG GAC GGG AGG CCT TTG YTG GAG; IgM 5'CAG GAG ACG AGG GGG AAA AG. Die klassenspezifische Differenzierung wird durch eine Reamplifikation der schweren Ketten in einer "nested" PCR erreicht, wobei erneut Amplifikationsprodukte gewonnen werden. In den "nested" PCR Reaktionen werden die Amplifikationsprodukte aus der PCR Reaktion als Matrize und 5' Primern, die sequenzkomplementär zu den ersten 6 Codons der V_{H} Region oder der V_{L} Regionen sind, und 3' Primern, die sequenzkomplementär zu den "genesteten" konstanten Regionen der κ-, λ-, α1-, α2-, γ1-, γ2-, γ3-, γ4-, µ- und ε- Ketten sind, zum Einsatz kommen, und so ein Amplifikationsprodukt gewonnen wird. Als "sense" primer werden die "sense" primer der PCR Reaktion verwendet. Als anti-"sense" Primer werden dabei nachfolgende Immunglobulin-klassenspezifische Oligonukleotide eingesetzt: IgA1 5'GAAAAC CCA GCT CAG CCC AAA CTC CAT; IgA2 5' CCT TGG GGC TGG TCG GGG ATG; IgE 5' CAG GAC GAC TGT AAG ATC TTC ACG; IgG1 5' CCC CAG AGG TGC TCT TGG AGG AGG GT; IgG2 5' CCG CTG TGC TCT CAG AGG TGC TCC TG; IgG3 5' GGC CGC TGT GCC CCC AGA GGT GCT C; IgG4 5' GCA GCC CAG GGC GGC TGT GCT CTC G; IgM 5'GAA AAG GGT TGG GGC GGA TGC. Die Amplifikationsreaktionen werden mit Taq Polymerase durchgeführt und die erhaltenen Fragmente, die V_{L} und V_{H} Regionen enthalten, werden in Vektoren kloniert. Insbesondere wird dabei das TA Klonierungssystems verwendet. Der vierte Schritt des Verfahrens zur Bestimmung des immunologischen Reaktionsprofils eines Organismus ist die rekombinante Herstellung von Antikörpern durch Exprimierung der Amplifikate. Dabei werden die in Vektoren klonierten Fragmente in bakteriellen und/oder eukaryontischen Expressionssystemen exprimiert. Die Vektoren werden zur Herstellung von rekombinanten Antikörpern verwendet. Insbesondere werden die resultierenden Immunglobulinfragmente als "single-chain Antikörper" oder als "Fab- Fragmente" zunächst in Phagen exprimiert. Die so erhaltenen Antikörper werden verwendet um ihre Bindungsaktivität zu Allergenen zu analysieren. Dabei wird die Bindungsaffinität der "single-chain Antikörper" mittels "biosensor chip" Technologie analysiert. Für die Expression der Fab-Fragmente als lösliche Proteine wird das unter Beispiel 1 beschriebene Expressionssystem verwendet.

Für die Messung der Bindungsaffinität wurde als Antigen rekombinantes Betvl an einen CM5 sensor chip kovalent gekoppelt. Weiter wurden zwei sequenzierte schwere und leichte Ketten, sowie eine schwere Kette allein in ein geeignetes Phagenexpressionssystem kloniert. Anschließend wurden zwei Fab-exprimierende Phagen injiziert. Das Experiment zeigt zwei Fab-Fragmente unterschiedlicher Affinität (Fab. high, Fab low). Die Spezifität ist durch die beiden Kontrollen (F-heavy und vector) gewährleistet. Das Experiment zeigt eindeutig, dass mit der beschriebenen Methode affine antigenspezifische B-Zellen isoliert werden können.

Das Verfahren eignet sich somit für die Bestimmung des Potentials für atopische Erkrankungen oder Antikörper vermittelte Autoimmunerkrankungen, sowie auch zur Bestimmung des immunologischen Reaktionsprofils von Personen mit bereits offensichtlichen Immunfunktionsstörungen. Zu den Immunfunktionsstörungen, die mittels vorliegender Erfindung analysierbar sind, werden nachfolgende Erkrankungen gerechnet: Antiglomeruläre Basalmembrankrankheit; Arthritis, rheumatoide; Autoimmunkrankheiten des Nervensystems, z.B. Multiple Sklerose; Diabetes mellitus, insulinabhängiger; Lupus erythematodes, systemischer; Pemphigus; Addison-Krankheit; Anämie, autoimmunhämolytische; Antiphospholipid-Syndrom; Dermatitis, herpetiformis Duhring; Glomerulonephritis, IgA-; Glomerulonephritis, membranöse; Goodpasture-Syndrom; Graves-Krankheit; Lambert-Eaton-Myasthenie-Syndrom; Ophthalmie, sympathische; Pemphigoid, bullöses; Polyendokrinopathien, Autoimmun-; Purpura, thrombozytopenische, idiopathische; Reiter-Krankheit; Thyreoiditis, Autoimmun; Hashimoto Thyreoiditis; primäres Myxödem; Thyreotoxikose (Basedow Krankheit); Myasthenia gravis; Anämie, perniziöse; Leukopenie, autoimmune; Thrombozytopenie, idiopathische; Zirrhose, primäre, biliäre; Autoimmunhepatitis; Colitis ulcerosa; Sjörgen Syndrom; rheumatisches Fieber; Dermatomyositisl Polymyositis; Sklerose, progressive, systemische; Granulomatose, Wegner; Panateriitis nodosa; Periateriitis nodosa; und Hypersensitivitätsangiitis usw. Das Verfahren erlaubt, das genetisch ermittelte Reaktionsprofil mit Reaktionsprofilen aus dem Serum qualitativ und quantitativ zu vergleichen. Nachstehend werden Ergebnisse diskutiert, die sich auf die in den Beispielen beschriebenen Experimente beziehen.

### Isolation antigenspezifischer B-Zellen

Ausgangspunkt für die Selektionssschritte stellen Leukozytenpräparationen aus Ficollgradientenzentrifugationen dar, auf der Basis von "buffy coats" bzw. peripherem Vollblut. Der Anteil CD19⁺ Zellen betrug 10,6 ± 0,6% bei einer Gesamtzellzahl von 3,4 ± 0,7 x 10⁷ PBMC.

Die Separation antigenspezifischer B-Zellen wurden mittels MACS-Verfahren (Miltenyi) durchgeführt. In einem zweifach gestaffelten Selektionsverfahren wurden zunächst CD19⁺-Zellen isoliert und aus dieser Fraktion antigen-spezifisch markierte Zellen. Deren Anteil variierte zwischen 0,01 und 0,05%. Die Zellen wurden im Anschluss EBV-transformiert.

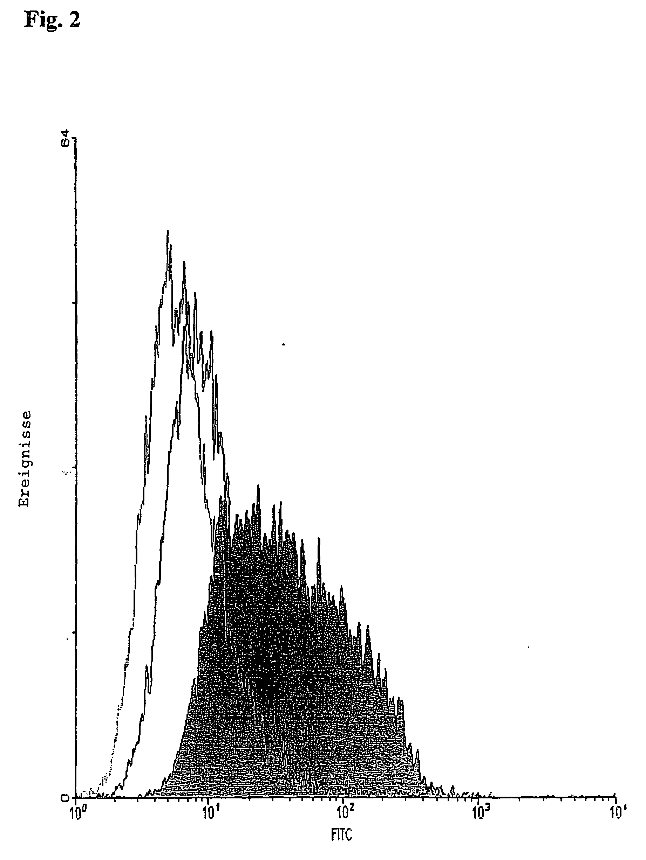

Die Bindungseigenschaften der kultivierten antigenspezifisch selektionierten B-Zellen wurden durchflusszytometrisch analysiert. Als Antigen diente ein rekombinantes Fusionsprotein (PV 2¹/₂ Ang II); bestehend aus dem eigentlichen Antigen PV 2¹/₂, einem Fragment des Lieschgras-Allergens rPhl pVb gekoppelt an humanes Angiogenin (Ang II). Als weiteres Antigen wurde das Fusionsprotein LPV-ETA' eingesetzt, eine Fusion des Vollängen-Allergens mit der Toxindomäne ETA'. Um nicht-Antigen-spezifische Reaktionen - im Sinne der vorangegangen Selektion gegen das Allergen - von Reaktionen unterscheiden zu können, die sich gegen Epitope der Toxindomänen richten, wurden Versuche mit rekombinanten Proteinfusionen durchgeführt, bei denen die Allergendomäne gegen rekombinante scFv - spezifisch für die Oberflächenantigene GD2 (NAng GDII) bzw. den Epidermalen Wachstumsfaktor- Rezeptor (HAI-ETA') -ausgetauscht waren. Im Gegensatz zu PV 2¹/₂ Ang II und LPV-ETA' zeigte der Einsatz beider Kontrollantigene in der FACS-Analyse Fluoreszenzintensitäten, die dem Hintergrundniveau der sekundären Nachweiskomponenten entsprachen (Figur 1 a und Figur 2 a). Mit einem weiteren Versuchsansatz sollte abgeklärt werden, ob die eingesetzten Proteine generell B-Zellen binden oder auf Eigenschaften spezifischer B-Zellen zurückzuführen sind. Dazu wurde derselbe Versuchsansatz vor dem Hintergrund eines großen B-Zell Rezeptorrepertoires mit frisch präparierten PBMCs durchgeführt und die Bindungsaktivität der CD19⁺ Fraktion untersucht (Figur 1 b). Für alle Allergen- und Kontrollvarianten ergeben sich identische Fluoreszenzsignalerkennungen und damit keinerlei Hinweise auf unspezifische Bindungen durch B-Zellen.

Zur durchflusszytometrischen Selektion Antigen-spezifischer B-Zellen wurden 4x10⁶ EBV-transformierte Zellen eingesetzt. Die Zellen waren ursprünglich als Antigen-spezifische Zellinie aus einer MACS-Selektion hervorgegangen. Mit zunehmenden Alter der Kultur wuchs der Anteil nicht bindender Zellen, vermutlich genetisch unstabile Klone, die keine funktionellen Immunglobuline oder B-Zellrezeptoren exprimieren konnten. Die verbliebenen Antigen-spezifischen Zellen ließen sich im Durchflusszytometer klar von unspezifischen unterscheiden und konnten über ein entsprechendes Selektions-"Gate" isoliert werden. Aus der Ursprungspopulation wurden 3x10⁵ Zellen, mit einer Reinheit von 97% isoliert und in Dichten von 1.000 Zellen pro Kavität in Kultur genommen. Nach 2 Wochen wurden die Antigen-spezifischen Bindungseigenschaften überprüft (Figur 2). Das Antigen (LPV) lag als Fusionsprotein vor, gekoppelt an die Toxinkomponente (ETA'). Um Reaktionen gegen die Toxindomäne erfassen zu können, wurden Kontrollversuche mit Hilfe eines Fusionskonstruktes (HAI-ETA') durchgeführt, bei dem die Antigendomäne durch rekombinantes EGF (epidermal growth factor) ersetzt war. Die Bindungen waren ausschließlich gegen die Antigendomäne (LPV) gerichtet. Die Fluoreszenzintensität des Kontrollantigens entsprach dem des Hintergrundes (sekundäre und tertiäre Nachweiskomponenten).

Um zu überprüfen, ob die Interaktionen zwischen Antigen und B-Zellen Eigenschaften spezifischer Klone im Sinne einer Antikörper/ Anitgenreaktion darstellen, wurden Antigen-spezifisch-selektioniert B-Zellen unselektionierten Zellinien gegenübergestellt. Die unselektionierten Zellen repräsentieren ein klonales B- Zellrepertoire aus einem "buffy coat", die durch EBV-Transformation immortalisiert und kultiviert wurden. Der Versuchsansatz entspricht dem oben beschriebenen, mit Ausnahme des Kontrollantigens. Gegenüber nicht-selektionierten Zellen B-Zellen wurden bei den Antigen-spezifisch selektionierter Zellen 4,1 fach höhere Bindungsaktivitäten (mittleren Fluoreszenzintensität) ermittelt.

### Immortalisierung durch EBV- Transformation

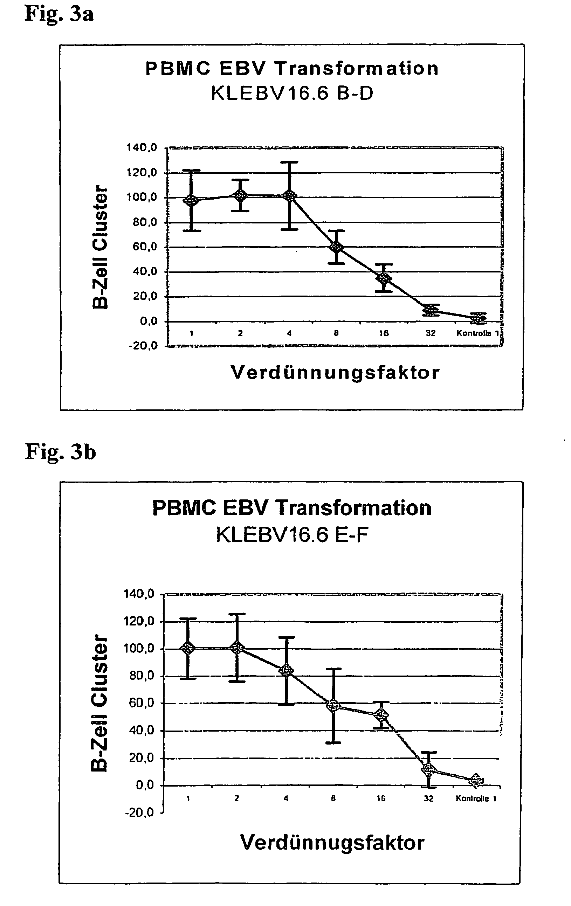

Zur Immortalisierung von primären B-Zellen wurden Leukozytenpräparationen dem unter Beispiel 1.2 beschrieben Verfahren unterzogen. Zur Bestimmung deroptimaler Transformationsparameter wurde Transformationsfrequenz EBV-haltiger Oberstände mit nicht selektionierten PBMCs ermittelt. Bei konstanter Zellzahl (1x10⁵) wurden Transformationsansätze mit kontinuierlichen 2-fach Verdünnungen des EBV-haltigen Oberstands bei zwei verschiedenen PBMC Präparationen durchgeführt. Nach 17 tägiger Kultivierungphase konnten transformierte B-Zellen als cluster-förmig wachsenden Zellaggregate beobachtet werden. Zur Vereinfachung wurde jeder Cluster als einzelner Klon gewertet. Die Anzahl der Cluster wurde in Bezug zur Ausgangszellzahl und zur jeweiligen Verdünnungsstufe der EBV-haltigen Oberstände gesetzt (Figur 3). Die Transformationsfrequenzen betrugen für die PBMC Präparation A 3,1 x 10⁻³ ± 1,7x 10⁻³ und für die Präparation B 3,5 x 10⁻³ ± 2,4 x 10⁻³.

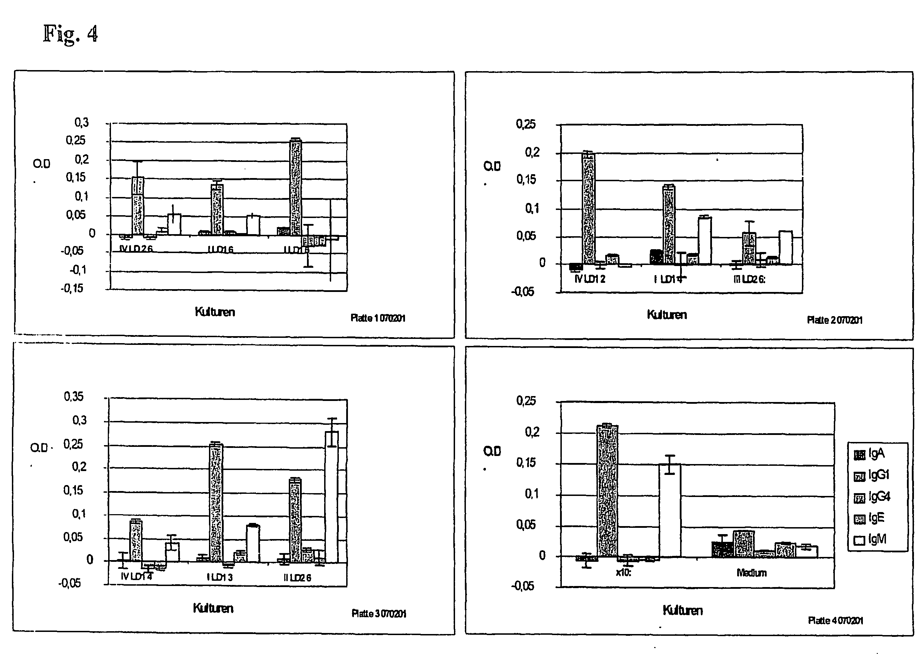

Um zu klären welche Immunglobulinklassen die in Dauerkultur befindlichen Selektionskulturen sezernieren wurden Kulturüberstände mittels Festphasen ELISA getestet. Die Untersuchung umfasste humane Antikörper der Klassen IgA, IgE, IgG1, IgG4 und IgM. Bei allen getesteten Kulturen konnten sezernierte Anitkörper nachgewiesen werden, die ausschließlichen den Klassen IgG1 und IgM zuzuordnen waren (Figur 4).

### Lymphozyten Isolation mittels Ficoll- Dichtegradienten Zentrifugation

Der Separation Antigen-spezifischer B-Zellen geht eine Leukozytenpräparation mittels Ficollgradientenzentrifugation voran (siehe auch Beispiel 1). Ausgangsmaterial für die Präparation stellt peripheres Vollblut aus Patienten dar. Allergikern wird 10 ml periphäres Vollblut in Heparin-beschichtete Blut-Röhrchen abgenommen. Es folgt eine Dichtegradienten Zentrifugation. Der Überstand enthält das ca. 1:3 verdünnte Serum und kann für ELISA auf -20°C gelagert werden. Die festen Blutbestandteile (Erythrozyten, Thrombozyten etc.) sinken bis zum Boden des Probenröhrchens, die Lymphozyten erscheinen als Ring zwischen Serum und Ficoll Lösung und werden mittels Pipette abgenommen. Man wäscht die Lymphozyten in einem Volumen von 30ml FACS Puffer (1 X PBS, 0.5% BSA, 4mM EDTA) bei 4°C für 20 min bei 1500 rpm mit Bremse und resuspendiert das Lymphozyten-Pellet in 1 ml FACS Puffer.

### B-Zellisolation mittels CD19-MACS Anreicherung

Die Lymphozyten werden mit einem anti-human CD19-MACS-Antikörper (Milteny) inkubiert (siehe ebenfalls Beispiel 1). Nach zweimaligem Waschen werden bis zu 2 x 10⁸ Gesamt-Zellen auf die MS-MACS Separations Säule (Miltenyi) geladen. Zuvor wird die Säule mit 500 µl FACS Puffer äquilibriert und anschließend 3 x mit 500 µl FACS Puffer gewaschen. Die Säule wird danach vom Magnet entfernt, mit 1 ml FACS Puffer überschichtet und die angereicherten Zellen werden vorsichtig mit dem Spritzenstempel durchgedrückt. Die Reinheit kann auf 99% erhöht werden, wenn man die positive, CD19⁺-angereicherte Fraktion über eine zweite, äquilibrierte Säule laufen lässt.

### Isolierung antigenspezifischer Gedächtniszellen

Hochaffine Antikörper werden von Antigenspezifischen B-Zellen in "Reifungszentren (germinal centers)" sekundär lymphatischer Organe mittels somatischer Mutation nach Antigenkontakt gebildet. Deshalb konzentriert sich die weitere Anreichung von CD19⁺-Populationen auf die Isolierung von Gedächtniszellen (memory cells). Für die Anreicherung von Gedächtniszellen werden standardmäßig publizierte anti-CD-Antikörper (Oberflächenmarker) verwendet. Die Spezifität der B-Zellpopulation für das Antigen wird dadurch erreicht, dass das Antigen mit einem Fluoreszenzmarker versehen wird (z.B. FITC). Die Fluoreszenz der Zellen wird nach der Färbung im FACS-Vantage Durchflußzytometer dargestellt. Zellen deren Signale die maximale Fluoreszenzintensität des Kontrollansatzes um eine Zehnerpotenz übertreffen, werden über ein Selektions-"Gate" erfasst und über den Einzelzell-Sortierungsmodus im 96 Weil Plattenformat abgelegt.

### RNA-Isolierung, cDNA-Synthese und RT-PCR Birkenpollen-reaktiver Immunglobulin schwerer und leichter Ketten

Die positiven Zellen werden sofort in eine 96-well Platte sortiert, in die 20 µl 1x RT-PCR Puffer (QIAGEN oneStep RT-PCR Kit) vorgelegt wurden. Die RNA Isolierung erfolgt nach dem Quiagen Standardprotokoll. Für die reverse Transkription (RT) werden antisense-spezifische Primer aus der 3'-Region von der konstanten Region der kappa- und lambda-leichten Kette, sowie der isotypspezifischen schweren Kette (epsilon (ε), alpha (α), mü (µ) und gamma (γ)-Subklassen) verwendet (die Sequenzen sind vollständig in der Sequenzdatenbank vorhanden). Es folgt der PCR-Amplifikationsschritt, bei dem ein Aliquot des RT- Ansatzes mit den jeweiligen sense primern aus der 5'-variablen Region von der kappa- und lambda leichten Kette, sowie der variablen schweren Kette zugefügt wird. Als Primer werden hierfür standardmäßig publizierte V-Gen Mixe von schweren und leichten Ketten verwendet. Die PCR- Bedingungen wurden für folgende Zeiten und Temperaturen optimiert (45 s 95°C, 45 s 72°C, 1 min 59°C; 35 repeats). Um die Spezifität der PCR zu erhöhen kann ein semi-nested PCR-Protokoll angewendet werden. Die resultierenden PCR-Produkte werden direkt sequenziert (ABI). Aufgrund der erhaltenen Sequenz wird das PCR-Produkt in geeignete Expressionsvektoren umkloniert.

### Expression rekombinanter Birkenpollen-reaktiver Immunglobulin schwerer und leichter Ketten.

Die Expression der schweren und leichten Ketten als scFv oder Fab-Fragmente erfolgt wie in Beispiel 1 beschrieben.

### Immortalisierung antigenspezifischer B-Lymphozyten mittels EBV-Transformation

Die Immortalisierung wird mittels EBV- Transformation erreicht. Die Methode ist in Beispiel 1 genau erläutert.

### BEISPIELE

### 1. Isolation Lieschgraspollen-reaktiver rekombinanter Antikörper und-fusionen

### 1.1. Isolation Antigen-spezifischer B-Zellen

Die Separation Antigen-spezifischer B-Zellen wurde mittels MACS-Verfahren durchgeführt (Abb. 1). Als Antigen diente Biotin-konjugiertes Lieschgraspollen-Allergen (rPhl pVb). Die Markierung immortalisierter Zellen erfolgte mit 2 µg Antigen/10⁶ Zellen, einem Waschschritt und einer anschließenden Inkubation mit Strepavidin-gekoppelten paramagnetischen Mikrobeads. Die markierten Lymphozyten wurden durch Retention im Magnetfeld von nicht markierten Zellen getrennt und Kultur genommen.

Alternativ zur MACS Isolation wurden spezifische B-Zellen mittels Durchflusszytometrie gegen Phl-pVb (FACSVantage SE, Becton Dickinson) selektioniert. Zur Markierung spezifischer Zellen wurde eine Fusion des rekombinanten Allergens PhI pVb mit der Toxindomäne des *Pseudomonas* Exotoxins A (ETA), LPV-ETA; eingesetzt (2 µg LPV-ETA' auf 1x10⁶ Zellen in 300 µl, 30'/4°C). Das Fusionsprotein ist über ein Poly-Histidintag detektierbar. Als Nachweisantikörper dienten monoklonale anti-His Antikörper (Arbeitskonzentration 1: 2000) und Ziege anti-Maus FITC Konjugat (1:1000). Die Fluorezenz der Zellen wurde nach der Färbung im FACSVantage SE Durchflusszytometer im FL 1/ FL2 dargestellt. Zellen deren Signale die maximale Fluoreszenzintensität des Kontrollanssatzes um eine Zehnerpotenz übertrafen wurden über ein Selektions-"Gate" erfasst und über den Einzelzell-Sortierungsmodus im 96 Well Plattenformat in eine Zellkulturplatte abgelegt. Die selektionierten Zellen wurden in Dauerkultur genommen und auf spezifische Bindungsaktivität getestet

### 1.2. Immortalisierung EBV- Transformation

Zur Gewinnung EBV-haltiger Überstände wurden kryokonservierte B-95-8 Zellen (CATCC CLR 1612) in einer Konzentration von 1 x 10⁶ Zellen in 10 ml Medium (RPMI 1640 10% FKS, 50 µg/ml Streptomycin, 50 U/ml Penicillin, 2 mM L-Glutamin) in Kultur genommen (37 °C, 5% CO₂). Nach Zugabe von 5 ml Kulturmedium am folgenden Tag und weiterer 10-tägiger Kultivierung ohne Mediumzugabe wurden die Zellkulturüberstände über einen 0,2 µm-Filter filtriert, aliquotiert und bei -196°C eingefroren.

Zur Transformation wurden periphere mononukleäre Zellen (PBMC) eingesetzt, die über Dichtegradienten- Zentrifugation aus Vollblut oder buffy coats' gewonnen wurden und anschließend einem, der unter 1.1 beschriebenen, Selektionsverfahren unterzogen wurden. Der Transformationsansatz setzte sich aus jeweils 100 µl Zellsuspension (10⁵ Zellen), 10 µl Cyclosporin A (2,6 µg/ml final) und 100 µl EBV-Überstand zusammen. Nach 17-tägiger Kultivierung (37 °C, 5% CO₂) ohne Mediumwechsel konnten immortalisierte B-Zellen anhand der entstandenen Zellaggregate beobachtet und in Kultur genommen werden. Der Zellkulturüberstand wurde mit Hilfe eines ELISA auf sezernierte Immunglobuline untersucht (Abb. 3). Die Detektion erfolgte mittels spezifischer Antikörper gegen humane Immunglobulinklassen (lgA, IgG1, IgG4, IgE und IgM), sowie Biotin-konjugierter anti Ziege-, bzw. anti Maus-Antikörper und alkalische Phosphatase-gekoppeltem Streptavidin. Als Negativkontrolle wurde das Kulturmedium (RPMI 1640, 10% FKS) eingesetzt und jeweils ein Ansatz ohne primäre Nachweiskomponente. Die untersuchten Überstände entstammten vier Monate alten Kulturansätzen nach vorausgegangener Subkultivierung Immunglobulin-sezernierender Zellen via "limiting dilution" (125 Zellen / Kavität).

### 1.3. Expression rekombinanter, bindungsfähiger V-Gen Fragmente

Bindungsfähige, einzelsträngige variable Fragmente (scFv) wurden mittels Standardverfahren [6] in ein Derivat des Phagemids pCANTAB oder den bakteriellen Expressionsvektor pBM1.1 (7) kloniert und dadurch mit einer Deletionsmutante des Pseudomonas Exotoxin A fusioniert. Die Ligationsansätze wurden phenolextrahiert, ethanolpräzipitiert und in 10 µl H2O resuspendiert. 2.5 µl dieser DNA-Lösung wurde nach einem beschriebenen Protokoll [7] durch Elektroporation in *E.coli* BL21 (DE3) eingebracht. Transformierte Bakterien wurden auf Kanamycin-haltigem Medium (2xLB mit 50 µg Kanamycin/ml und 2% Glukose) kultiviert. Positive Klone wurden durch Restriktionsanalyse verifiziert.

Die Verfahren zur Klonierung und Expression der scFV sind bereits beschrieben unter

| **Scfv** | **Zielantigen** | **Erkrankung** | **Referenzen** |
|---|---|---|---|
| Ki-4 | CD30-Rezeptor | Hodgkin-Lymphom | [8, 9] |
| Ki-3 | CD30-Rezeptor | Hodgkin-Lymphom | [10, 11]] |
| hHAK30 | CD30-Rezeptor | Hodgkin-Lymphom | [12] |
| RFT5 | CD25-Rezeptor | Hodgkin-Lymphom | [13-15] |
| 14.18 | GD2-Antigen | Neuroblastom | [13, 14] |

### 1.4. Anzucht und Induktion der gentechnisch veränderten Organismen

Transformierte E.coli BL21 (DE3) wurden in Terrific Broth-Medium [TB] [6]; 1,2% Bacto-Trypton, 2,4% Bacto-Yeast Extract, 0,4%, 0,17 M KH₂PO₄, 0,72 M K₂HPO₄) supplementiert mit 50 µg/ml Kanamycin und 0,5 mM ZnCl₂ für 12-15 h bzw. über Nacht bei 26°C und 200 rpm inkubiert. Anschließend wurden die Kulturen in 0,5 1-Erlenmeyerkolben mit Schikanen, in einem Volumen von 200 ml TB-Medium überführt und bis zu einer OD₆₀₀ von 2 angezogen. Anschließend wurde der osmotische Wert des Mediums mit 0,5 M Sorbitol, 4% NaCl heraufgesetzt und als kompatibles Solut 10 mM Betain oder Ectoin hinzugefügt. Beide Substanzen haben vergleichbare Wirkung, wobei allerdings Ectoin über Kotransformation mit Plasmiden der pOSM-Reihe oder durch zusätzliche Kodierung auf dem pBM1.1- Derivat auch direkt von den Zellen synthetisiert werden kann.

Die Kulturen wurden für 15 - 60 min bei 26°C/200 rpm inkubiert und dann wurde durch Zugabe von 2 mM IPTG die Expression des Targetproteins eingeleitet. Nach Erreichen einer OD₆₀₀ von 2,3 - 2,4 wurden die Bakterien durch einen Zentrifugationsschritt (3.700 g, 10 min, 4°C) geerntet. Alle weiteren Schritte wurden auf Eis durchgeführt. Nach einem Waschschritt (Pellet wurde in 75 mM Tris/HCl pH 8, 4% NaCl, 10% Glycerin resupendiert und homogenisiert) mit anschließender Zentrifugation (3.700 g, 10 min, 4°C) wurde das Gewicht des Naßpellets bestimmt und das Bakterienpellet vor dem Aufschluss bei -80°C bzw. bei - 196°C für 10 min schockgefroren.

Ein alternativer Ansatz zur Expression rekombinanter scFv wird nach folgenden Verfahren durchgeführt: Transformierte E.coli TG1 werden gepickt und in 2 x TY, 100 µg/ml Ampicillin, 2% Glukose aufgenommen und über Nacht bei 37°C unter Schütteln inkubiert. Anschließend wird die über Nacht inkubierte Kultur 1:100 in 50 ml 2 x YT Broth-Medium, 100 µg/ml Ampicillin, 2% Glukose verdünnt und bei 37°C unter Schütteln auf einen Dichte von 0,9 OD₆₀₀ₙₘ angezogen. Der Ansatz wird bei 4.000 rpm 10 Minuten zentrifugiert und das Pellet in 10 ml 2xTY, 100 µg/ml Ampicillin, 1 mM ITPG resuspendiert. Unter kontinuierlichem Schütteln bei 30°C wird der Ansatz für mindestens 5 weitere Stunden inkubiert.

Das Konstrukt kann nach 5 Stunden Induktion aus dem Periplasma abgeerntet werden. Dazu wird die Kultur für 10 bis 20 Minuten auf Eis gekühlt und im Anschluss in einer Microfuge bei 6.000 rpm für 5 Minuten zentrifugiert. Die Pellets werden in 10% des ursprünglichen Volumens in PBS, 1 M NaCl, 1 mM EDTA 4°C aufgenommen und danach für 30 Minuten auf Eis inkubiert. Es folgt ein weiterer Zentrifugationsschritt in der Mikrofuge für 10 Minuten bei 6.000 rpm um die Zellen zu entfernen. Der Überstand wird in ein frisches Tube überführt und bei 14.000 rpm, 4°C für 10 Minuten zentrifugiert um Debris zu sedimentieren. Im Überstand befindet sich die periplasmatische Protein Fraktion.

### 1.5. Aufschluss und Aufreinigung rekombinanter scFv und scFv-Fusionen

Im Anschluss an die Einfrierphase wurde das Bakterienpellet in Ultraschallpuffer (75 mM Tris/HCl pH 8, 300 mM NaCl, 1 Tablette Proteaseinhibitoren [Boeringer]/50 ml, 5 mM DTT, 10 mM EDTA, 10% Glycerol) auf Eis resuspendiert, homogenisiert (15 min) und anschließend 6 x 30 s bei 200 Watt Ultraschall- behandelt. Zwischen den einzelnen Inkubationsphasen erfolgte eine gleich lange Abkühlungsphase auf Eis (30 s). Um die scFv-Fusionen (Immuntoxine) quantitativ zu gewinnen, war es nötig, das Pellet nochmals bei -80°C bzw. -196°C einzufrieren und die soeben beschriebenen Arbeitsschritte maximal 3x zu wiederholen. Die gewonnenen Fraktionen wurden vereinigt und sterilfiltriert. Nach sequentieller 50% Ammoniumsulfat (AS)-Fällung wurden die Proben in 30% AS für 4-6 h inkubiert, anschließend in 50 % AS angereichert und schließlich bei 4°C über Nacht präzipitiert. Zur Gewinnung der Proben werden diese bei 25.000 g für 45 min zentrifugiert. Das Proteinpellet wurde in Probenpuffer (75 mM Tris/HCl pH 8, 300 mM NaCl, 10% Glycerol) aufgenommen. Danach wurden die Proben bei 4°C mittels Hitrap-Desalting Säule auf einer FPLC Anlage umgepuffert (75 mM Tris/HCl pH 8, 300 mM NaCl, 10% Glycerol). Im Anschluss an die Entsalzungssäule wurde das Protein in einem ersten Schritt metallchelatchromatographisch über eine Ni-NTA-Säule (Qiagen) aufgetragen.

### 2. Isolierung, Charakterisierung und Expression Birkenpollen spezifischer Antikörper aus Allergikern

### 2.1 Lymphozyten Isolation mittels Ficoll- Dichtegradienten Zentrifugation

Der Separation Antigen-spezifischer B-Zellen geht eine Leukozytenpräparation mittels Ficollgradientenzentrifugation voran (siehe auch Beispiel 1). Ausgangsmaterial für die Präparation stellt peripheres Vollblut aus Patienten dar. Allergikern wird 10 ml periphäres Vollblut in Heparin-beschichtete Blut-Röhrchen abgenommen. Es folgt eine Dichtegradienten Zentrifugation. Der Überstand enthält das ca. 1:3 verdünnte Serum und kann für ELISA auf -20°C gelagert werden. Die festen Blutbestandteile (Erythrozyten, Thrombozyten etc.) sinken bis zum Boden des Probenröhrchens, die Lymphozyten erscheinen als Ring zwischen Serum und Ficoll Lösung und werden mittels Pipette abgenommen. Man wäscht die Lymphozyten in einem Volumen von 30ml FACS Puffer (1 X PBS, 0.5% BSA, 4mM EDTA) bei 4°C für 20 min bei 1500 rpm mit Bremse und resuspendiert das Lymphozyten-Pellet in 1 ml FACS Puffer.

### 2.2 B-Zelllsolation mittels CD19-MACS Anreicherung

Die Lymphozyten werden mit einem anti-human CD19-MACS-Antikörper (Milteny) inkubiert (siehe ebenfalls Beispiel 1). Nach zweimaligem Waschen werden bis zu 2 x 10⁸ Gesamt-Zellen auf die MS-MACS Separations Säule (Miltenyi) geladen. Zuvor wird die Säule mit 500 µl FACS Puffer äquilibriert und anschließend 3 x mit 500 µl FACS Puffer gewaschen. Die Säule wird danach vom Magnet entfernt, mit 1 ml FACS Puffer überschichtet und die angereicherten Zellen werden vorsichtig mit dem Spritzenstempel durchgedrückt. Die Reinheit kann auf 99% erhöht werden, wenn man die positive, CD19⁺-angereicherte Fraktion über eine zweite, äquilibrierte Säule laufen lässt.

### 2.3 Isolierung antigenspezifischer Gedächtniszellen

Hochaffine Antikörper werden von Antigenspezifischen B-Zellen in "Reifungszentren (germinal centers)" sekundär lymphatischer Organe mittels somatischer Mutation nach Antigenkontakt gebildet. Deshalb konzentriert sich die weitere Anreichung von CD19⁺-Populationen auf die Isolierung von Gedächtniszellen (memory cells). Für die Anreicherung von Gedächtniszellen werden standardmäßig publizierte anti-CD-Antikörper (Oberflächenmarker) verwendet. Die Spezifität der B-Zellpopulation für das Antigen wird dadurch erreicht, dass das Antigen mit einem Fluoreszenzmarker versehen wird (z.B. FITC). Die Fluoreszenz der Zellen wird nach der Färbung im FACS-Vantage Durchflußzytometer dargestellt. Zellen deren Signale die maximale Fluoreszenzintensität des Kontrollansatzes um eine Zehnerpotenz übertreffen, werden über ein Selektions-"Gate" erfasst und über den Einzelzell-Sortierungsmodus im 96 Weil Plattenformat abgelegt.

### 2.4 RNA-Isolierung, cDNA-Synthese und RT-PCR Birkenpollen-reaktiver Immunglobulin schwerer und leichter Ketten

Die positiven Zellen werden sofort in eine 96-well Platte sortiert, in die 20 µl 1x RT-PCR Puffer (QIAGEN oneStep RT-PCR Kit) vorgelegt wurden. Die RNA Isolierung erfolgt nach dem Quiagen Standardprotokoll. Für die reverse Transkription (RT) werden antisense-spezifische Primer aus der 3'-Region von der konstanten Region der kappa- und lambda-leichten Kette, sowie der isotypspezifischen schweren Kette (epsilon (ε), alpha (α), mü (µ) und gamma (γ)-Subklassen) verwendet (die Sequenzen sind vollständig in der Sequenzdatenbank vorhanden). Es folgt der PCR-Amplifikationsschritt, bei dem ein Aliquot des RT- Ansatzes mit den jeweiligen sense primern aus der 5'-variablen Region von der kappa- und lambda leichten Kette, sowie der variablen schweren Kette zugefügt wird. Als Primer werden hierfür standardmäßig publizierte V-Gen Mixe von schweren und leichten Ketten verwendet. Die PCR- Bedingungen wurden für folgende Zeiten und Temperaturen optimiert (45 s 95°C, 45 s 72°C, 1 min 59°C; 35 repeats). Um die Spezifität der PCR zu erhöhen kann ein semi-nested PCR-Protokoll angewendet werden. Die resultierenden PCR-Produkte werden direkt sequenziert (ABI). Aufgrund der erhaltenen Sequenz wird das PCR-Produkt in geeignete Expressionsvektoren umkloniert.

### 2.5 Expression rekombinanter Birkenpollen-reaktiver Immunglobulin schwerer und leichter Ketten.

Die Expression der schweren und leichten Ketten als scFv oder Fab-Fragmente erfolgt wie in Beispiel 1 beschrieben.

### 2.6 Immortalisierung antigenspezifischer B-Lymphozyten

Die Immortalisierung wird mittels EBV- Transformation erreicht. Die Methode ist in Beispiel 1 genau erläutert.

### Literatur

1. Mudde, G.C., et al., Antigen presentation in allergic sensitization. Immunol Cell Biol, 1996. 74(2) : p. 167-173.
2. Vercelli, D., et al., To E or not to E? Can an IL-4-indiuced B cell choose between IgE and IgG4? Int Arch Allergy Immunol, 1998. 116(1): p. 1-4.
3. Lieby, P., et al., The clonal analysis of anticardiolipin antibodies in a single patient with primary antiphospholipid syndrome reveals an extreme antibody heterogeneity. Blood , 2001. 97(12): p. 3820-3828.
4. Wang, X. and B.D. Stollar, Human immunoglobulin variable region gene analysis by single cell RT-PCR. J Immunol Methods, 2000. 244(1-2): p. 217-225.
5. Luger E., Lamers M., Achatz-Straussberger G., Geisberger R., Inführe D., Breitenbach M., Crameri R. and Achatz G. Somatic diversity of the immunoglobulin repertoire is controlled in an isotype-specific manner. (2001) Eur. J. Immunol. 31: 2319-2330
6. Sambrook J., Fritsch E.F. and Maniatis T. (1989) Molecular Cloning, Alaoboartory Manual, second edition CSH Cold Spring Harbor laboratory Press, ISBN 0-87969-309-6
7. Dower WJ, Miller JF, Ragsdale CW. High efficiency transformation of E. coli by high voltage electroporation. Nucleic Acids Res. 1988 Jul 11;16(13):6127-45.
8. Klimka, A., et al., An anti-CD30 single chain Fv selected by phage display and fused to Pseudomonas exotoxin A (Ki-4(scFv)-ETA') is a potent immunotoxin against Hodkin-derived cell line- Br J Cancer, 1999. 80(8): p. 1214-1422.
9. Barth, S., et al., (Ki-4(scFv)-ETA'), a new recombinant anti-CD30 immuntoxin with highly specific cytotoxic activity against disseminated Hodkin tumors in SCID mice. Blood, 2000. 95(12): p. 3909-3914.
10. Matthey, B., et al., Recombinant immunotoxins for the treatment of Hodkin's disease (Review). Int J Mol Med, 2000. 6(5): p. 509-514.
11. Hansen, H.P., et al., Inhibition of metalloproteinases enhances the internalization of anti-CD30 antibody Ki-3 and the cytotoxic activity of Ki-3 immunotoxin. Int J Cancer, 2002. 98(2): p. 210-215.
12. Klimka, A., et al., Human anti-CD30 recombinnat antibodies by guided phage antibody selection using cell panning. Br J Cancer, 2000. 83(2): p. 252-260.
13. Tur, M.K., et al, Selection of scFv phages on intact cells under low pH conditions leads to a significant loss of insert-free phages. Biotechniques, 2001. 30(2): p. 410, 412-413.
14. Barth, S., et al., Construction and in vitro evaluation of RFT5(scFv)-ETA', a new recombinant single-chain immunotoxin with specific cytotoxicity towards CD25+-Hodkins-derived cell lines. Int J Mol med, 1998. 1(1): p. 249-256.
15. Barth, S., et al., Recombinnat anti-CD25 immunotoxin RFT5(scFv)-ETA' demonstrates successful elimination of disseminated human Hodkin lymphoma in SCID mice. Int J Cancer, 2000. 86(5): p. 718-724.
13. Luger, E., et al., Somatic diversity of the immunoglobuline repertoire is controlled in an isotype-specific manner. Eur J Immunol, 2001. 31(8) : p. 2319-2330.

## Patentansprüche

1. Verfahren zur Bestimmung des antigenspezifischen V-Gen Repertoires aller Immunglobulinklassen eines Organismus *in vitro,* umfassend die folgenden Schritte:
(a) Isolation, einmalig oder mehrmalig, einer Fraktion aus Körperflüssigkeiten und/oder Geweben, die B-Zellen enthält;
(b) Isolation von einzelnen B-Zellen oder deren genetischen Materials und Ablage von einer oder mehreren B-Zellen oder deren genetischen Materials in ein Reaktionsgefäß wobei die Isolation einzelner B-Zellen die folgenden Schritte umfasst:
(i) die Markierung der B-Zellen mittels Fluorochrom-gekoppelter Antikörper spezifisch für CD19, CD85 und CD138, CD19, CD85, CD 138, CD10, CD20, CD21, CD22, CD23, CD24, CD27, CD37, CD38, CD39, CD40, CD72, CD73, CD74, CDw75, CDw76, CD77, CDw78, CD79, CD80, CD81, CD82, CD83, CDw84, CD85 und/oder CD86,
(ii) die durchflusszytometrische Selektion der markierten B-Zellen und
(iii) die Ablage einer oder mehrerer B-Zellen in ein Reaktionsgefäß.
(c) Amplifikation des die variablen Regionen eines von einer isolierten B-Zelle exprimierten Antikörpers codierenden genetischen Materials unter Berücksichtigung aller Immunglobulinklassen, umfassend die folgenden Schritte:
(i) cDNA generierende RT Reaktionen, wobei Primer eingesetzt werden, die sequenzkomplementär zu den konstanten Regionen der leichten und schweren Ketten sind, und als Amplifikationsprodukt ein cDNA Produkt gewonnen wird,
(ii) PCR Reaktionen mit der cDNA als Matrize, wobei degenerierte Primer, die sequenzkomplementär zu den variablen Regionen im framework 1 Bereich der 6 Genfamilien der schweren Ketten sind und/oder PCR Reaktionen mit degenerierten Primern für die 4 Genfamilien der κ-Ketten und/oder PCR Reaktionen mit degenerierten Primern für die 3 Genfamilien der λ-ketten und/oder PCR Reaktionen mit degenerierten Primern für die konstanten Regionen der ε- γ- und der κ-Ketten eingesetzt werden, wobei Amplifikationsprodukte gewonnen werden, und
(iii) nested PCR Reaktionen mit den Amplifikaten aus der PCR Reaktion als Matrize und 5' Primern, die sequenzkomplementär zu den ersten 6 Codons der V_{H} Region oder der V_{L} Regionen sind, und 3' Primern, die sequenzkomplementär zu den "genesteten" konstanten Regionen der κ-, λ-, α1-, α2-, γ1-, γ2-, γ3-, γ4-, µ- und ε- Ketten sind, wobei Amplifikationsprodukte gewonnen werden;
(d) Klonierung der Amplifikationsprodukte in einen Vektor;
(e) Herstellung von rekombinanten Antikörpern durch Exprimierung der klonierten Amplifikationsprodukte; und
(f) Bestimmung der Bindung des rekombinanten Antikörpers an rekombinante und/oder native Antigene.

2. Verfahren nach Anspruch 1 zur Bestimmung des Immunologischen Reaktionsprofils eines Organismus, dessen . Immunsystem nicht offensichtlich gestört ist.

3. Verfahren nach Anspruch 2, wobei es sich bei dem Organismus um eine Person handelt.

4. Verfahren nach Anspruch 2 und/oder 3, wobei das Potential einer Prädisposition für atopische Erkrankungen oder Antikörper vermittelte Autoimmunerkrankungen bestimmt wird.

5. Verfahren nach Anspruch 1 zur Bestimmung des immunologischen. Reaktionsprofils eines Organismus mit Immunfunktionsstörungen.

6. Verfahren nach Anspruch 5, wobei es sich bei dem Organismus um eine Person handelt.

7. Verfahren nach Anspruch 1, wobei die B-Zellen nach dem Isollerungsschritt und der Einzeizellablage durch EBV-Transformation immortalisiert werden.

8. Verfahren nach Anspruch 1, wobei die B-Zellen nach dem Isolierungsschritt und der Einzellzellablage mit Myelomzellen fusioniert werden.

9. Verfahren nach Anspruch 1, wobei die Bestimmung der Bindung der rekombinanten Antikörper an Antigene durch automatisierte Verfahren erfolgt.

10. Verfahren nach Anspruch 9, wobei die Bestimmung der Bindung der rekombinanten Antikörper an Antigene durch festphasengekoppelte Bindungsassays, Durchflußzytometrie, Resonanzspektrometrie und/oder Chip Arrays mit rekombinanten und nativen Antigenen erfolgt.

## Claims

1. A process for determining the antigen-specific V-gene repertoire of all immunoglobulin classes of an organism *in vitro,* comprising the following steps:
(a) isolation of a fraction of body fluids and/or tissues containing B cells, performed once or several times;
(b) isolation of individual B cells or of the genetic material thereof and placing one or more B cells or their genetic material in a reaction vessel, the isolation of individual B cells comprising the following steps:
(i) labeling the B cells by fluorochrome-coupled antibodies specific for CD19, CD85 and CD138, CD19, CD85, CD 138, CD10, CD20, CD21, CD22, CD23, CD24, CD27, CD37, CD38, CD39, CD40, CD72, CD73, CD74, CDw75, CDw76, CD77, CDw78, CD79, CD80, CD81, CD82, CD83, CDw84, CD85 and/or CD86;
(ii) selecting the labeled B cells by flow cytometry; and
(iii) placing one or more B cells in a reaction vessel;
(c) amplification of the genetic material coding for the variable regions of an antibody expressed by an isolated B cell involving all immunoglobulin classes, comprising the following steps:
(i) cDNA-generating RT reactions using primers whose sequence is complementary to the constant regions of the light and heavy chains to obtain a cDNA product as the amplification product;
(ii) PCR reactions with the above cDNA as a template using degenerate primers whose sequence is complementary to the variable regions in the framework 1 region of the 6 gene families of the heavy chains, and/or PCR reactions using degenerate primers for the 4 gene families of the κ chains, and/or PCR reactions using degenerate primers for the 3 gene families of the λ chains, and/or PCR reactions using degenerate primers for the constant regions of the ε, γ and κ chains, to obtain amplification products; and
(iii) nested PCR reactions with the amplificates from the PCR reaction as the template and 5' primers whose sequence is complementary to the first 6 codons of the V_{H} region or V_{L} regions, and 3' primers whose sequence is complementary to the nested constant regions of the κ, λ, α1, α2, γ1, γ2, γ3, γ4, µ and ε chains, to obtain amplification products;
(d) cloning the amplification products into a vector;
(e) producing recombinant antibodies by expressing the cloned amplification products; and
(f) determining the binding of the recombinant antibody to recombinant and/or native antigens.

2. The process according to claim 1 for determining the immunological reaction profile of an organism whose immune system is not obviously disordered.

3. The process according to claim 2, wherein said organism is a human.

4. The process according to claim 2 and/or 3, wherein the potential of a predisposition for atopical diseases or antibody-mediated autoimmune diseases is determined.

5. The process according to claim 1 for determining the immunological reaction profile of an organism with immunodeficiency.

6. The process according to claim 5, wherein said organism is a human.

7. The process according to claim 1, wherein said B cells are immortalized by EBV transformation after the steps of isolating and placing the individual cells.

8. The process according to claim 1, wherein said B cells are fused with myeloma cells after the steps of isolating and placing the individual cells.

9. The process according to claim 1, wherein the determination of the binding of the recombinant antibodies to antigens is effected by automated methods.

10. The process according to claim 9, wherein the determination of the binding of the recombinant antibodies to antigens is effected by solid-phase coupled binding assays, flow cytometry, resonance spectrometry and/or chip arrays with recombinant and native antigens.

## Revendications

1. Procédé de détermination du répertoire de gènes V spécifique de l'antigène de toutes les classes d'immunoglobuline d'un organisme *in vitro,* comprenant les étapes suivantes :
(a) isolement, unique ou répété, d'une fraction provenant de fluides corporels et/ou de tissus qui contient des cellules B ;
b) isolement de cellules B individuelles ou de leur matériau génétique et dépôt d'une ou de plusieurs cellules B ou de leur matériau génétique dans un réacteur, l'isolement de cellules B individuelles comprenant les étapes suivantes:
(i) le marquage des cellules B au moyen d'anticorps couplés à des fluorochromes, spécifiques pour CD19, CD85 et CD138, CD19, CD85, CD138, CD10, CD20, CD21, CD22, CD23, CD24, CD27, CD37, CD38, CD39, CD40, CD72, CD73, CD74,CDw75,CDw76, CD77, CDw78, CD79, CD80, CD81, CD82, CD83, CDw84, CD85 et/ou CD86,
(ii) la sélection par cytométrie en flux des cellules B marquées et
(iii) le dépôt d'une ou de plusieurs cellules B dans un réacteur.
(c) amplification du matériau génétique codant pour les régions variables d'un anticorps exprimé par une cellule B isolée, avec prise en compte de toutes les classes d'immunoglobuline, comprenant les étapes suivantes:
(i) des réactions RT génératrices d'ADNc, des amorces étant utilisées qui sont complémentaires en séquence aux régions constantes des chaînes légères et lourdes, et un produit ADNc étant obtenu en tant que produit d'amplification,
(ii) des réactions PCR avec l'ADNc en tant que matrice, des amorces dégénérées qui sont complémentaires en séquence aux régions variables dans la région d'ossature 1 des 6 familles de gènes des chaînes lourdes, et/ou des réactions PCR avec des amorces dégénérées pour les 4 familles de gènes des chaînes κ, et/ou des réactions PCR avec des amorces dégénérées pour les 3 familles de gènes des chaînes λ, et/ou des réactions PCR avec des amorces dégénérées pour les régions constantes des chaînes ε, γ et κ étant utilisées, des produits d'amplification étant obtenus, et
(iii) des réactions PCR imbriquées avec les amplificats issus de la réaction PCR en tant que matrice et des amorces 5' qui sont complémentaires en séquence aux 6 premiers codons de la région V_{H} ou des régions V_{L}, et des amorces 3' qui sont complémentaires en séquence aux régions constantes "nichées" des chaînes κ, λ, α1, α2, γ1, γ2, γ3, γ4, µ et ε, des produits d'amplification étant acquis ;
(d) clonage des produits d'amplification dans un vecteur ;
(e) fabrication d'anticorps recombinants par expression des produits d'amplification clonés ; et
(f) détermination de la liaison de l'anticorps recombinant avec des antigènes recombinants et/ou natifs.

2. Procédé selon la revendication 1 pour la détermination du profil de réaction immunologique d'un organisme dont le système immunitaire n'est apparemment pas détérioré.

3. Procédé selon la revendication 2, l'organisme étant une personne.

4. Procédé selon la revendication 2 et/ou la revendication 3, le potentiel d'une prédisposition à des maladies atopiques ou à des maladies auto-immunes transmises par des anticorps étant déterminé.

5. Procédé selon la revendication 1 pour la détermination du profil de réaction immunologique d'un organisme présentant des détériorations des fonctions immunitaires.

6. Procédé selon la revendication 5, l'organisme étant une personne.

7. Procédé selon la revendication 1, les cellules B étant immortalisées par transformation EBV après l'étape d'isolement et de dépôt individuel.

8. Procédé selon la revendication 1, les cellules B étant fusionnées avec des cellules du myélome après l'étape d'isolement et de dépôt individuel.

9. Procédé selon la revendication 1, la détermination de la liaison des anticorps recombinants avec des antigènes étant effectuée par des procédés automatisés.

10. Procédé selon la revendication 9, la détermination de la liaison des anticorps recombinants avec des antigènes étant effectuée par des essais de liaison couplés en phase solide, par cytométrie en flux, par spectrométrie de résonance et/ou par des réseaux de puces avec antigènes recombinants et natifs.
